# EUROPEAN PATENT APPLICATION

(11) **EP 4 389 901 A1**
(43) Date of publication of application: **26.06.2024**
(21) Application number: 22858727.5
(22) Date of filing: 17.08.2022
(51) Int. Cl.: C12N 15/70

(54) **METHOD FOR STABLY MAINTAINING POLY (A) TAIL**

(30) Priority: 17.08.2021 KR 20210108264
(71) Applicant: SK Bioscience Co., Ltd., Bundang-gu, Seongnam-si, Gyeonggi-do, 13494 (KR)
(72) Inventor: SHIN, Jin Hwan, Seongnam-si, Gyeonggi-do 13494 (KR); CHOI, Young Joon, Seongnam-si, Gyeonggi-do 13494 (KR); KIM, Hun, Seongnam-si, Gyeonggi-do 13494 (KR); KWON, Yong-min, Seongnam-si, Gyeonggi-do 13494 (KR); JEONG, Hye-won, Seongnam-si, Gyeonggi-do 13494 (KR)
(74) Representative: Vossius & Partner Patentanwälte Rechtsanwälte mbB
(86) International application number: PCT/KR2022/012233
(87) International publication number: WO 2023/022490

(57) **Abstract**

The present invention relates to a method for stably maintaining a poly(A) tail.

## Description

### [Technical Field]

The present invention relates to a method for stably maintaining a poly(A) tail.

### [Background Art]

mRNA has recently been attracting attention as an active pharmaceutical ingredient (API). mRNA requires a component called a poly(A) tail, which plays an important role in the stability of RNA and the translation process. In the case of mRNA constituting an mRNA vaccine, the length of the poly(A) tail is recognized as having a standard size of 120 *(*Mol Ther Nucleic Acids. 2021 Dec 3;26:945-956). mRNA transcribed within cells has a poly(A) signal and undergoes the process of adding a poly(A) tail after the transcription process. mRNA produced by *in vitro* transcription (IVT) also requires a poly(A) tail to increase the stability in the body and the translation efficiency.

There are two methods to add a poly(A) tail. One method is to synthesize a poly(A) tail using poly(A) polymerase after IVT, and another method is to contain an adenine repeat sequence in the template used for IVT so that a poly(A) tail is generated through IVT.

In the former case, a separate process is required in order to synthesize a poly(A) tail in mRNA using poly(A) polymerase, and the length of the generated poly(A) tail may not be constant. However, in the latter case, a separate process other than the IVT process is not required, and the length of the generated poly(A) tail may be constant. Therefore, in terms of process efficiency and uniformity of poly(A) tail length, it may be preferable to contain the poly(A) tail sequence in the template.

To achieve this, it is necessary to clone and maintain the poly(A) tail sequence in plasmid DNA, but recombination and deletion occur when this process is performed using *E. coli* in a general manner, and it is difficult to obtain plasmid DNA containing 100 or more adenine repeat sequences, which is the standard size for use in mRNA vaccines.

In order to solve this problem, a method has been designed to increase the stability by containing a linker sequence between adenine repeat sequences (RNA. 2019 Apr;25(4):507-518. doi: 10.1261/rna.069286.118. Epub 2019 Jan 15.), but it is still required that other methods be developed for cloning and maintaining a poly(A) tail in a plasmid without containing a linker sequence between the poly(A) tails since a poly(A) tail consisting only of adenine repeat sequences is the original form that exists in nature.

### [Disclosure]

### [Technical Problem]

An object of the present invention relates to a method for stably maintaining a poly(A) tail.

### [Technical Solution]

The present invention provides a method for stably maintaining a poly(A) tail.

### [Advantageous Effects]

Through the method of the present invention, a poly(A) tail can be easily cloned, and a poly(A) sequence can be stably maintained.

### [Brief Description of Drawings]

FIG. 1 illustrates colony PCR results when a poly(A) sequence is cloned using DH5alpha under a condition of 37°C;
FIG. 2 illustrates colony PCR results when a poly(A) sequence is cloned using Stbl3 under a condition of 37°C;
FIG. 3 illustrates colony PCR results when a poly(A) sequence is cloned under a low-temperature condition;
FIG. 4 illustrates a graph showing the results of comparing the A124±1 ratios when cell lines transformed with a plasmid containing a sequence encoding a poly(A) tail are cultured under various temperature conditions; and
FIG. 5 illustrates graphs showing the results of culture curves at the respective temperatures acquired by measuring OD₆₀₀ at predetermined times while prepared stocks are cultured at 200 rpm for 48 hours under conditions of 13°C, 16°C, 19°C, 22°C, and 25°C.

### [Detailed Description of the Invention]

An aspect embodying the present invention is a method for stably maintaining a poly(A) tail in a plasmid.

In an embodiment, the method includes culturing a cell line transformed with a plasmid containing a sequence encoding a poly(A) tail under a temperature condition of 31°C or less.

In any one of the above-described embodiments, the culturing step includes (1) recovering the cell line under a temperature condition of 31°C or less.

In any one of the above-described embodiments, the method includes (2) culturing the cell line on a solid medium under a temperature condition of 31°C or less to form a colony after the recovery step of (1).

In any one of the above-described embodiments, the culture further includes (3) culturing the colony formed by the solid medium culture of (2) in a liquid medium under a temperature condition of 31°C or less.

In any one of the above-described embodiments, the method includes (1) recovering a cell line transformed with a plasmid containing a sequence encoding a poly(A) tail under a temperature condition of 31°C or less; (2) forming a colony through solid culture of the recovered cell line under a temperature condition of 31°C or less; and (3) performing liquid culture under a temperature condition of 31 °C or less.

In any one of the above-described embodiments, the step of culturing the colony in a liquid medium includes performing subculture.

In any one of the above-described embodiments, the temperature condition in the steps (1) to (3) is 16°C to 31°C

In any one of the above-described embodiments, the temperature condition is in the steps (1) to (3) 19°C to 25°C.

In any one of the above-described embodiments, the cell line is *E*. *coli.*

In any one of the above-described embodiments, the transformation includes introducing a plasmid containing a sequence encoding a poly(A) tail into a cell line using heat shock or electroporation.

In any one of the above-described embodiments, the introduction is performed at a temperature of 37°C to 42°C.

In any one of the above-described embodiments, the method further includes preserving the cultured cell line.

In any one of the above-described embodiments, the preserving step includes freezing and/or drying the cell line.

In any one of the above-described embodiments, the culture includes culturing and activating the preserved strain.

In any one of the above-described embodiments, the method increases stability of a poly(A) tail in a plasmid compared to a method including culturing a cell line transformed with a plasmid at a temperature of 37°C or more.

In any one of the above-described embodiments, the poly(A) tail contains 20 to 400 adenines (A).

Another aspect embodying the present invention is a method for mass-producing a plasmid for mRNA production.

In an embodiment, the method includes culturing a cell line transformed with a plasmid containing a sequence encoding an mRNA containing a poly(A) tail under a temperature condition of 31 °C or less.

In any one of the above-described embodiments, the mRNA is a component of an immunogenic composition and/or a therapeutic agent.

Another aspect embodying the present invention is a method for producing an mRNA containing a poly(A) tail.

In an embodiment, the method includes culturing a cell line transformed with a plasmid containing a sequence encoding an mRNA containing a poly(A) tail under a temperature condition of 31°C or less.

In any one of the above-described embodiments, the method includes recovering a plasmid from a cell line and performing transcription *in vitro.*

In any one of the above-described embodiments, the mRNA is a component of an immunogenic composition and/or a therapeutic agent.

Hereinafter, the present disclosure will be described in more detail. Meanwhile, each description and embodiment disclosed in the present invention may also be applied to other descriptions and embodiments. That is, all combinations of various elements disclosed in the present invention fall within the scope of the present invention. Further, the scope of the present invention is not limited by the specific description below.

Further, those skilled in the art will recognize, or be able to ascertain using no more than routine experimentation, many equivalents to the specific embodiments of the present invention described herein. Further, these equivalents should be interpreted to fall within the scope of the present invention.

Additionally, a number of papers and patent documents are referenced and citations are indicated throughout this specification. The disclosures of the cited papers and patent documents are incorporated herein by reference in their entirety to more clearly explain the contents of the present invention and the level of technical field to which the present invention pertains.

An aspect of the present invention is a method for stably maintaining a poly(A) tail in a plasmid.

The method includes culturing a cell line transformed with a plasmid containing a sequence encoding a poly(A) tail under a temperature condition of 31°C or less.

In the present invention, the term "poly(A) tail" refers to a continuous or discontinuous sequence of adenylate residues typically located at the 3' end of an RNA molecule. The poly(A) tail may follow the 3', for example, the 3'UTR of mRNA. Such a poly(A) tail may consist of or contain about 20 or more, 25 or more, 40 or more, 60 or more, 80 or more, or 100 or more adenyl (A) nucleotides.

In an embodiment, the poly(A) tail may contain 20 to 400 adenines (A). For example, the poly(A) tail may contain 20 to 300, 40 to 200, 50 to 190, 60 to 180, 70 to 170, 60 to 160, 70 to 150, or 80 to 140 adenines. For example, the poly(A) tail may contain about 120 adenines, but is not limited thereto.

In an embodiment, based on the number of nucleotides of the poly(A) tail, typically at least 75%, at least 80%, at least 85%, at least 90%, at least 95%, at least 96%, at least 97%, at least 98%, or at least 99% are A nucleotides, but the remaining nucleotides may be nucleotides other than A nucleotides, for example, U, T or C. As an example, the poly(A) tail may contain modifications that delay degradation of mRNA.

In the present invention, a plasmid containing a poly(A) tail may contain a promoter capable of initiating transcription, any operator sequence for regulating such transcription, a sequence encoding a desired mRNA, and sequences that regulate the termination of transcription and translation. In an embodiment, the plasmid may contain a restriction enzyme binding site that is easy to use for cloning.

In an embodiment, the plasmid may contain a selection marker to confirm transformation. Markers that confer selectable phenotypes such as drug resistance, auxotrophy, and resistance to cytotoxic agents may be used, and in an environment treated with a selective agent, only cells expressing the selectable markers survive or show other expression traits, so transformed cells can be selected. However, the plasmid is not limited to the above-mentioned examples.

The plasmid of the present invention can be produced by introducing a sequence encoding a poly(A) tail into a plasmid.

As an example, the sequence encoding a poly(A) tail may be amplified by PCR, treated with a restriction enzyme, and then linked to a restriction enzyme-treated plasmid to prepare a plasmid containing a sequence encoding a poly(A) tail. As an example, a polynucleotide sequence encoding an mRNA may be linked to the top of the sequence encoding a poly(A) tail. As an example, the plasmid may be used as a template for transcribing a desired mRNA, which contains a polynucleotide sequence encoding the mRNA at the top of the sequence encoding a poly(A) tail.

The cell line transformed with a plasmid containing a sequence encoding a poly(A) tail of the present invention may be prepared by introducing a plasmid containing a sequence encoding a poly(A) tail into a cell line.

In the present invention, introducing a plasmid containing a sequence encoding a poly(A) tail into a cell line may also be referred to as transforming the cell line with the plasmid.

In the present invention, "introduction" or "transformation" of a plasmid means delivering the plasmid to a cell line. Such introduction may be easily performed in accordance with conventional methods in the art. In order to introduce a plasmid into a cell line, cells may be treated so as to be permeable to DNA molecules, and the cells that have undergone this process are called competent cells.

In an embodiment, the introduction may be performed using heat shock or electroporation.

Plasmid delivery using heat shock is performed by mixing DNA and cells and momentarily applying heat. As an example, the introduction by heat shock may be performed under a temperature condition of about 37°C or more, specifically under a temperature condition of about 37°C to 42°C. Chilling the cells on ice before and/or after application of heat shock may be included.

Electroporation is a method of delivering a plasmid using electricity, and when a short, high-voltage electric pulse is applied to change the membrane potential of the cell membrane, nanometer-sized pores are formed on the surface of the cell membrane and DNA permeability increases. As an example, the introduction by electroporation may be performed under a temperature condition of about 37°C or more, specifically under a temperature condition of about 37°C to 42°C. Chilling the cells on ice before and/or after application of electrical stimulation may be included.

As another example, a CaCl₂ precipitation method, the Hanahan method in which a reducing substance called DMSO (dimethyl sulfoxide) is used in the CaCl₂ method to increase efficiency, a calcium phosphate precipitation method, a protoplast fusion method, a stirring method using silicon carbide fibers, a transformation method using PEG, and a dextran sulfate, lipofectamine, and drying/inhibition-mediated transformation method may be used. However, the introduction is not limited thereto.

In the case of mRNA for therapeutic purposes such as mRNA vaccines, the poly(A) tail is stably required for mRNA stability and translation efficiency, and the present applicant newly provides a method for culturing *E. coli* cloned by a method that enables stable cloning, mass production, and maintenance without loss of the sequence encoding the poly(A) tail present in the plasmid.

In the present invention, the step of culturing a cell line into which a plasmid containing a sequence encoding a poly(A) tail has been introduced under a temperature condition of 31°C or less may include any one or more of the following steps:
(i) recovering the cell line,
(ii) culturing the cell line on a solid medium, and
(iii) culturing the cell line in a liquid medium.

The step (i) of recovering the cell line is a step in which the cell line in a competent state after transformation is cultured in a medium to recover the physiological function. As an example, the medium in the recovery step may be a non-selective medium.

As an example, the culture temperature in the recovery step may be about 31°C or less, for example, about 16°C to 31°C, about 16°C to 30°C, about 16°C to 29°C, about 16°C to 28°C, about 16°C to 27°C, about 16°C to 26°C, about 16°C to 25°C, about 17°C to 25°C, about 18°C to 25°C, or about 19°C to 25°C.

As an example, the culture time in the recovery step may be about 5 minutes to 2 hours.

Colonies may be formed by culturing the transformed cell line through the step (ii) of culturing the cell line on a solid medium.

A "colony" refers to a population of cell lines into which a desired plasmid has been introduced.

A "solid medium" may also be referred to as "solid type medium." By solid medium culture, it is possible to examine whether the strain is growing and whether a plasmid has been introduced, and a selective medium may be used to examine whether a plasmid has been introduced. As an example, the selective medium may contain an antibiotic.

As an example, the temperature in the step of culturing the cell line on a solid medium may be about 31°C or less, for example, about 16°C to 31°C, about 16°C to 30°C, about 16°C to 29°C, about 16°C to 28°C, about 16°C to 27°C, about 16°C to 26°C, about 16°C to 25°C, about 17°C to 25°C, about 18°C to 25°C, or about 19°C to 25°C.

Mass culture of the cell line is possible through the step (iii) of culturing the cell line in a liquid medium. The step of culturing the cell line in a liquid medium may be culturing a single colony obtained in the step (ii) of culturing the cell line on a solid medium.

As an example, the temperature in the step of culturing the cell line in a liquid medium may be about 31°C or less, for example, about 16°C to 31°C, about 16°C to 30°C, about 16°C to 29°C, about 16°C to 28°C, about 16°C to 27°C, about 16°C to 26°C, about 16°C to 25°C, about 17°C to 25°C, about 18°C to 25°C, or about 19°C to 25°C.

As an example, the step of culturing the cell line in a liquid medium may include performing subculture. Subculture means transplanting some cells from the original culture into a fresh medium and culturing the cells anew. The cell density can be appropriately adjusted through subculture. As an example, the subculture may be performed two or three or more times, but is not limited thereto, and can be adjusted appropriately by those skilled in the art.

The method of the present invention may further include preserving the cultured cell line.

The preserving step includes freezing and/or drying the cell line. As the cryopreservative, for example, substances, such as glycerol or dimethyl sulfoxide, known in the art may be used.

In any one of the above-described embodiments, the culture includes culturing and activating the preserved strain. The preserved strain may be in a frozen or dried state. Culture in the activating step may be solid culture or liquid culture.

According to the method of the present invention, the poly(A) tail in the plasmid can be stably maintained during the steps of preserving and activating the cultured cell line.

The medium used in the culturing step of the present invention may contain nutrients necessary for culturing the cell line. For example, a conventional medium containing carbon sources, nitrogen sources, phosphorus sources, inorganic compounds, amino acids, vitamins, and/or the like may be used. Additionally, in the culturing step, compounds such as ammonium hydroxide, potassium hydroxide, ammonia, phosphoric acid, and sulfuric acid may be added to the medium in an appropriate manner to adjust the pH of the medium.

The cell line of the present invention is not particularly limited, but may be *E. coli. E. coli* is a host cell widely used for mass production or cloning of DNA, and the cell line of the present invention may include not only wild-type *E*. *coli* but also mutant *E*. *coli* engineered to have characteristics advantageous for cloning. As an example, a strain in which RecA deleted may be used. However, the cell line is not limited thereto.

The poly(A) tail contained in a plasmid may become shorter by recombination, deletion or the like within a cell line. However, when cells are cultured by the method of the present invention, the length of a poly(A) tail contained in a plasmid can be stably maintained.

As an example, the method increases the stability of a poly(A) tail in a plasmid compared to a method including culturing a cell line into which a plasmid has been introduced at a temperature of 37°C or more. The culture may include one or more of (i) recovering the cell line, (ii) culturing the cell line on a solid medium, or (iii) culturing the cell line in a liquid medium.

Another aspect of the present invention is a method for mass-producing a plasmid for mRNA production.

The method includes culturing a cell line transformed with a plasmid containing a sequence encoding an mRNA containing a poly(A) tail under a temperature condition of 31°C or less.

The poly(A) tail, plasmid, cell line, culture, and temperature condition are as described above.

As an example, the produced plasmid may be used as a template to synthesize an mRNA *in vitro.* As an example, the method may further include recovering the plasmid from the cell. However, the method is not limited thereto.

As an example, an mRNA containing a poly(A) tail, which is synthesized using the plasmid as a template, may be used as a component of an immunogenic composition.

As an example, an mRNA containing a poly(A) tail, which is synthesized using the plasmid as a template, may be used as a component of a therapeutic agent.

Another aspect of the present invention is a method for producing an mRNA.

The method includes culturing a cell line transformed with a plasmid containing a sequence encoding an mRNA containing a poly(A) tail under a temperature condition of 31°C or less; and performing transcription using the plasmid of the cell line as a template.

The poly(A) tail, mRNA containing a poly(A) tail, plasmid, cell line, culture, and temperature condition are as described above.

As an example, the plasmid may be recovered from the cell line after the culturing step.

As an example, the transcription step may be performed *in vitro.*

As an example, the production method may further include formulating the produced mRNA. When the produced mRNA is formulated, commonly used diluents or excipients such as fillers, extenders, binders, wetting agents, disintegrants, and surfactants may be used. As an example, the production method may include producing the mRNA in a form of being contained in lipid nanoparticles. However, the method is not limited thereto.

As an example, the produced mRNA containing a poly(A) tail may be used as a component of an immunogenic composition.

As an example, the produced mRNA containing a poly(A) tail may be used as a component of a therapeutic agent.

### [Examples]

Hereinafter, the present invention will be described in more detail with reference to Examples and Experimental Examples. However, these Examples and Experimental Examples are for illustrative purposes only, and the scope of the present invention is not limited to these Examples and Experimental Examples.

### Comparative Example 1. Cloning using general strain under normal conditions

In order to secure plasmid DNA having a poly(A) sequence inserted, the commonly used DH5alpha strain and method were used.

### Preparation of poly(A) fragment and conjugate

In order to prepare a poly(A) fragment to be inserted into a plasmid, DNA oligos (custom-made by Cosmogenetech) consisting of sequences of SEQ ID NO: 1 and SEQ ID NO: 2 were mixed at a 1:1 molar ratio, and annealed by lowering the temperature by 1°C per minute from 95°C, and the gap was filled with Klenow large fragment (NEB) to construct a double-strand poly(A) DNA fragment. Afterwards, the double-strand poly(A) DNA fragment was cut with restriction enzymes Sacll and Hindlll (NEB) and then inserted into a plasmid (pSKBS01, SEQ ID NO: 3) cut with the same restriction enzymes using T4 DNA ligase to prepare a conjugate.

### Transformation (DH5alpha)

The prepared conjugate was transformed into *E*. *coli* DH5alpha (Enzynomics, CP010). The conjugate was added to *E. coli* for transformation on ice, and the mixture was allowed to stand for 30 minutes and then subjected to heat shock at 42°C for 30 seconds. Afterwards, the mixture was cooled on ice for 2 minutes, SOC medium was added, and the *E*. *coli* was allowed to recover at 37°C for 1 hour and then plated on an antibiotic selective solid medium. The medium on which *E. coli* was plated was cultured at 37°C to generate colonies.

### Confirmation of poly(A) sequence insertion

Colonies formed on the selective medium after transformation were subjected to colony PCR to determine the possibility of insertion of a poly(A) fragment, the plasmid was then isolated from the colony confirmed to have the possibility of fragment insertion, and the inserted sequence was confirmed through base sequence analysis.

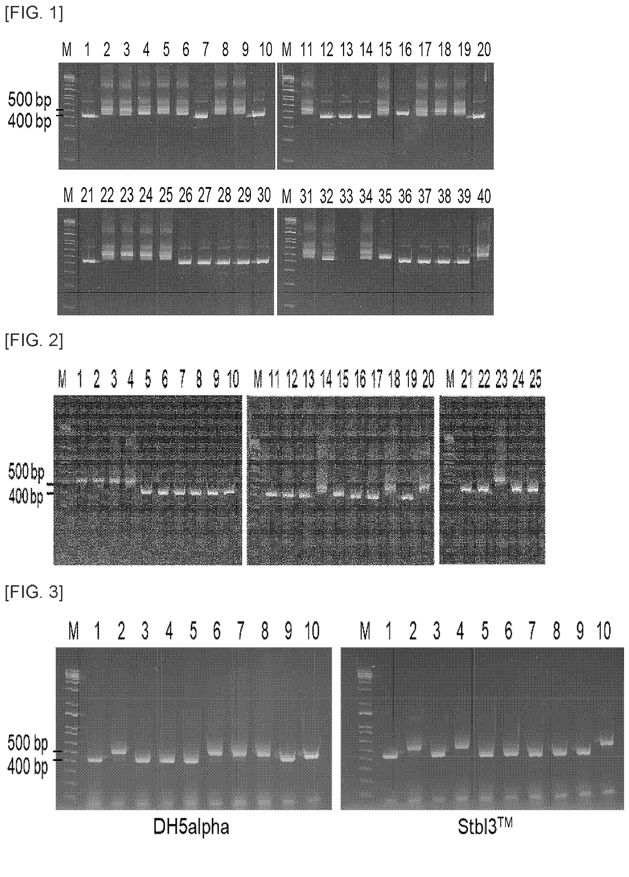

For colony PCR, M13-Forward and M13-Reverse primers (SEQ ID NOs: 4 and 5) were used, and it was controlled so that a PCR product of 550 bp was generated when poly(A) was inserted and a PCR product of 446 bp was generated when poly(A) was not inserted into the plasmid. PCR was performed on 40 colonies, and the PCR product was confirmed on agarose gel. As a result of colony PCR, 19 colonies clearly had 446 bp, indicating that poly(A) was not inserted, and the remaining 21 colonies had multiband patterns but appeared to contain a PCR product of 550 bp in size (FIG. 1). These 21 colonies, confirmed to have the possibility of fragment insertion, were subjected to liquid culture at 37°C, the plasmids were then isolated, and base sequence analysis was performed using Sanger sequencing.

However, it was confirmed that the poly(A) sequence was not inserted in all of the colonies subject to base sequence analysis.

This result supports that all poly(A) tails are lost during cloning under normal conditions.

### Comparative Example 2. Cloning using strain specialized for cloning unstable fragment

In order to secure plasmid DNA having a poly(A) sequence inserted, the Stbl3 strain, one of the strains specialized for cloning unstable fragments including repetitive sequences, was used. The culture temperature during transformation was 37°C, the same as that in Comparative Example 1.

### Transformation (Stbl3)

The conjugate prepared in Comparative Example 1 was transformed into *E. coli* Stbl3 (Invitrogen, C737303). The conjugate was added to *E. coli* for transformation on ice, the mixture was allowed to stand for 30 minutes and then subjected to heat shock at 42°C for 42 seconds. Afterwards, the mixture was cooled on ice for 2 minutes, SOC medium was added, the *E. coli* was allowed to recover at 37°C for 1 hour and then plated on an antibiotic selective solid medium. The medium on which *E*. *coli* was plated was cultured at 37°C to generate colonies.

### Confirmation of poly(A) sequence insertion

Colony PCR was performed in the same manner as in Comparative Example 1. PCR was performed on 25 colonies, and the PCR product was confirmed on agarose gel. Seventeen colonies clearly had 446 bp, indicating that poly(A) was not inserted. The remaining eight colonies had multiband patterns but appeared to contain a PCR product of 550 bp in size (FIG. 2). These eight colonies, confirmed to have the possibility of fragment insertion, were subjected to liquid culture at 37°C, the plasmids were then isolated, and base sequence analysis was performed using Sanger sequencing.

However, it was confirmed that the poly(A) sequence was not inserted in all of the colonies subject to base sequence analysis.

This result supports that the special strain that can stably clone unstable fragments also loses all poly(A) tails and is unstable to produce a stable mRNA vaccine.

### Example 1. Cloning using low-temperature condition

From the Comparative Examples described above, it was confirmed that there is a problem in that the poly(A) sequence is not properly inserted or stably maintained by conventional methods in which a poly(A) sequence is introduced into *E. coli* and then the *E*. *coli* is cultured at 37°C.

Accordingly, a method for stably maintaining a poly(A) tail was newly established. For this, the *E. coli* culturing temperature during transformation was decreased to be lower than the normal 37°C.

### Transformation (DH5alpha, Stbl3)

The conjugate prepared in Comparative Example 1 was transformed into *E. coli* DH5alpha (Enzynomics, CP010) and Stbl3 (Invitrogen, C737303), respectively. Conditions other than the *E*. *coli* culturing temperature were the same as those mentioned in Comparative Examples 1 and 2. After heat shock, the *E*. *coli* was allowed to recover at 25°C, plated on an antibiotic selective solid medium, and then cultured at 25°C to generate colonies.

### Confirmation of poly(A) sequence insertion

Colony PCR was performed in the same manner as in Comparative Example 1. PCR was performed on 10 colonies for each strain, and the PCR product was confirmed on agarose gel.

Five colonies in DH5alpha and seven colonies in Stbl3 clearly had 446 bp, indicating that poly(A) was not inserted, and PCR products of clear 550 bp in size rather than multiband patterns were observed in the remaining colonies unlike in Comparative Examples 1 and 2 (FIG. 3). These colonies, confirmed to have the possibility of fragment insertion, were subjected to liquid culture at 25°C, the plasmids were then isolated, and base sequence analysis was performed using Sanger sequencing.

As a result of the experiment, it was confirmed that the poly(A) sequence was inserted in all of the colonies subject to base sequence analysis.

Through this, it was confirmed that a low-temperature condition lower than 37°C is advantageous in order to stably clone a poly(A) tail in both general strains and strains specialized for cloning unstable fragments.

### Example 2. Exploration of temperature condition: transformation and sequence confirmation using general strain

In order to explore the temperature range in which a poly(A) tail can be stably maintained, plasmid DNA containing a poly(A) tail was transformed and subjected to liquid culture under various temperature conditions, and their base sequences were analyzed to confirm that the poly(A) tail was maintained.

### Transformation (DH5alpha)

Using the previously described method (Comparative Example 1), plasmid DNA having a poly(A) tail length of 124 was constructed, and the plasmid DNA was transformed into *E. coli* DH5alpha (Enzynomics, CP010). The plasmid DNA was added to *E. coli,* and the mixture was allowed to stand on ice for 30 minutes and then subjected to heat shock at 42°C for 40 seconds. Afterwards, the mixture was cooled on ice for 2 minutes, SOC medium was added, and the *E*. *coli* was cultured for 1 hour each at 37°C, 34°C, 32°C, 31°C, 28°C, 25°C, 22°C, and 19°C, allowed to recover, and then plated on an antibiotic selective solid medium. The medium on which *E*. *coli* was plated was cultured at the same temperature as the temperature at which *E*. *coli* recovered to generate colonies.

### Confirmation of length of poly(A) sequence

In order to isolate plasmids for base sequence analysis, 24 or 25 colonies generated on the solid medium were selected and subjected to liquid culture at the same temperature as the temperature at which the solid medium was cultured. After liquid culture, the plasmid was isolated, and the length and base sequence of the poly(A) sequence were analyzed using Sanger sequencing. The Atail-seq-R (SEQ ID NO: 6) primer was used for base sequence analysis.

As a result of base sequence analysis, it was confirmed that the poly(A) sequence is not maintained at 37°C, but the stability of poly(A) length is significantly increased when culture is performed at 31°C or less. It was thus confirmed that the stability of poly(A) sequence increases as the *E. coli* culturing temperature decreases.

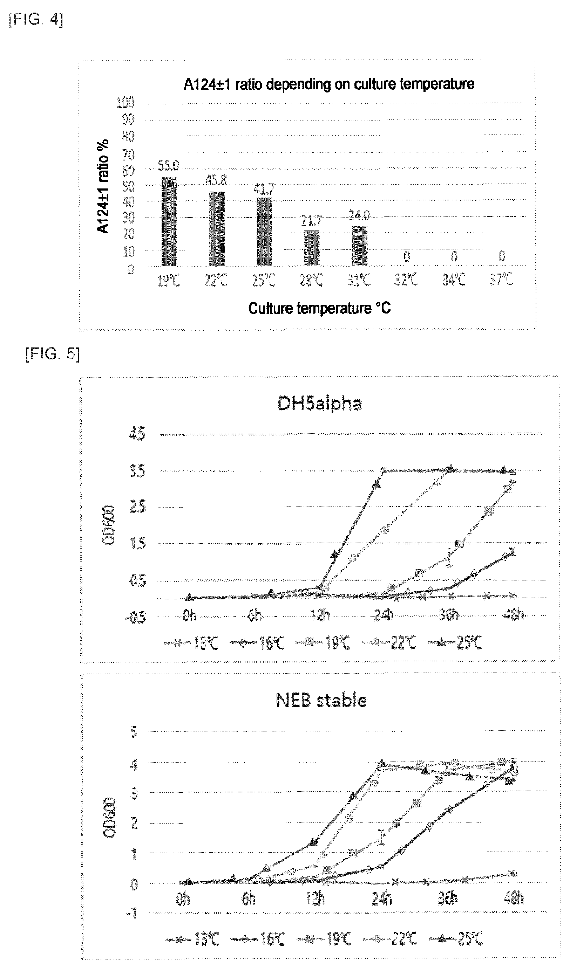

When a template having a long repeat of one sequence is analyzed in base sequence analysis using Sanger sequencing, one nucleotide may be omitted or added in the analysis result because of the slippage phenomenon of polymerase (https://www.nucleics.com/DNA sequencing support/DNA-sequencing-AT-slippage.html). Therefore, the ratio of A124±1, which is within 1 error range from the length of the inserted poly(A) sequence, was used as an indicator. The results are shown in Table 1 and FIG. 4.

**[Table 1]**

| Length of poly(A) sequence after transformation into DH5alpha at various temperatures | | | | | | | | |
|---|---|---|---|---|---|---|---|---|
| Colony # | Transformation and culture temperature | | | | | | | |
| | 19°C | 22°C | 25°C | 28°C | 31°C | 32°C | 34°C | 37°C |
| 1 | n/a | 123 | 121 | 26 | 123 | 19 | 19 | 19 |
| 2 | 124 | 92 | 124 | 123 | 15 | 17 | 19 | n/a |
| 3 | 122 | 122 | 117 | 15 | 121 | 17 | 19 | 15 |
| 4 | 19 | 102 | 124 | n/a | 123 | 19 | 19 | 19 |
| 5 | 122 | 124 | 122 | 122 | 19 | 9 | 9 | 19 |
| 6 | 123 | 124 | 122 | 124 | 121 | 15 | n/a | 15 |
| 7 | n/a | 124 | 57 | 19 | 122 | 16 | 17 | 24 |
| 8 | n/a | 124 | 122 | 121 | 15 | 19 | 15 | 15 |
| 9 | 19 | 120 | 124 | 122 | 123 | 17 | n/a | 15 |
| 10 | 124 | 122 | 125 | 123 | 19 | 15 | 19 | 15 |
| 11 | 124 | 124 | 123 | 123 | 123 | n/a | 15 | 19 |
| 12 | 123 | 124 | 124 | 121 | 112 | 19 | 19 | 18 |
| 13 | 122 | 90 | 123 | 121 | 19 | 121 | 122 | 17 |
| 14 | n/a | 119 | 106 | 19 | 123 | 15 | 19 | 20 |
| 15 | 124 | 123 | 124 | 15 | 15 | n/a | 19 | 20 |
| 16 | 123 | 123 | 19 | 122 | 122 | n/a | 19 | 18 |
| 17 | 122 | 35 | 108 | 122 | 19 | 19 | n/a | 19 |
| 18 | 125 | 122 | 66 | 19 | 19 | n/a | n/a | 17 |
| 19 | 123 | 123 | 44 | 19 | 123 | 19 | 19 | 17 |
| 20 | 123 | 15 | 17 | 15 | 120 | 27 | 15 | 18 |
| 21 | 123 | 19 | 120 | 122 | 121 | n/a | n/a | 13 |
| 22 | n/a | 15 | 124 | 119 | 122 | n/a | n/a | 36 |
| 23 | 121 | 123 | 124 | 123 | 19 | 19 | 15 | 17 |
| 24 | 15 | n/a | 122 | 122 | 19 | n/a | n/a | 16 |
| 25 | 19 | 15 | n/a | n/a | 106 | n/a | 19 | n/a |
| A124±1 ratio | **55.0%** | **45.8%** | **41.7%** | **21.7%** | **24.0%** | 0.0% | 0.0% | 0.0% |

| | | | | | | | | |
|---|---|---|---|---|---|---|---|---|
| *n/a: Mixed peak as result of Sanger sequencing and excluded from analysis | | | | | | | | |

As shown in Table 1 and FIG. 4, it can be seen that the A124±1 ratio is 21.7% to 55.0%, and the poly(A) tail is stably maintained in the plasmid when a cell line transformed with a plasmid containing a sequence encoding a poly(A) tail is cultured under a temperature condition of 31°C or less. However, since the growth of *E*. *coli* rarely occurs under a temperature condition of less than 16°C (Non-patent Document 1, Acta Alimentaria 50 (2021) 2, 180-188), the temperature condition of less than 16°C cannot be applied to the mass production process of vaccine products. It can be seen again in Example 5 described belwo that a temperature condition of less than 16°C is not appropriate for process application.

### Example 3. Stability during subculture: Transformation and subculture using strain specialized for maintaining unstable fragment

In order to examine whether a poly(A) sequence can be kept intact during continuous subculture under low-temperature conditions, transformation and subculture were performed at 37°C and 25°C using the NEB stable strain, one of the strains specialized for maintaining unstable fragments.

### Transformation (NEB stable)

The plasmid DNA used in Example 2 was transformed into *E*. *coli* NEB stable (NEB, C3040H). The plasmid DNA was added to *E. coli* for transformation on ice, and the mixture was allowed to stand for 30 minutes and then subjected to heat shock at 42°C for 40 seconds. Afterwards, the mixture was cooled on ice for 5 minutes, NEB 10-beta/Stable Outgrowth medium was added, and the *E. coli* was allowed to recover for 1 hour at 37°C or 25°C and then plated on an antibiotic selective solid medium. The medium on which *E. coli* was plated was cultured at the same temperature as the temperature at which *E*. *coli* recovered to generate colonies.

### Confirmation of length of poly(A) sequence after transformation

Among the colonies generated after transformation at each temperature, 25 colonies were selected and subjected to liquid culture at the same temperature as the temperature for solid culture. The plasmid was isolated and the length of the poly(A) sequence was analyzed using Sanger sequencing. The 3UTR-Xba1-F (SEQ ID NO: 7) primer was used for base sequence analysis. Compared to the results acquired from the commonly used DH5alpha in Example 2, it was confirmed that the ratio of colonies maintaining the length of poly(A) sequence of 120 or more during transformation at 37°C is relatively high. It is conceivable that these results are acquired by using a strain specialized for maintaining unstable fragments.

However, as can be seen through the A124±1 ratio, it was confirmed once again that a low temperature is advantageous for maintaining the length of the poly(A) sequence regardless of the strain as the number of colonies maintaining the number of poly(A) close to 124 originally contained in the plasmid used for transformation is significantly smaller at 37°C compared to that at 25°C.

**[Table 2]**

| Change in length of poly(A) sequence depending on temperature during transformation into NEB stable | | |
|---|---|---|
| Colony # | Culture temperature | |
| | 25°C | 37°C |
| 1 | 62 | 120 |
| 2 | 123 | 121 |
| 3 | 125 | 122 |
| 4 | 122 | 122 |
| 5 | 123 | 122 |
| 6 | 124 | 125 |
| 7 | 124 | 122 |
| 8 | 124 | 120 |
| 9 | 122 | 19 |
| 10 | 125 | 118 |
| 11 | 124 | 121 |
| 12 | 15 | 120 |
| 13 | 74 | 53 |
| 14 | 123 | 120 |
| 15 | 124 | 122 |
| 16 | 124 | 120 |
| 17 | 124 | 122 |
| 18 | 124 | 15 |
| 19 | 123 | 15 |
| 20 | 124 | 121 |
| 21 | 54 | 45 |
| 22 | 124 | 122 |
| 23 | 123 | 120 |
| 24 | 124 | 120 |
| 25 | 124 | 122 |
| A124±1 ratio | 76.00% | 4.00% |

### Subculture

Among the colonies in which the length of the poly(A) sequence was confirmed through base sequence analysis after transformation, six colonies were selected under a condition of 37°C and 9 colonies were selected under a condition of 25°C. The culture solution of the selected colonies was inoculated into a fresh liquid medium at a volume of 1/1000 and then subcultured at the same temperature. Subculture was carried out two times under a condition of 37°C and three times under a condition of 25°C.

### Confirmation of length of poly(A) sequence according to subculture

While subculture was carried out, plasmids were isolated from some of the culture solutions, and the length of the poly(A) sequence was analyzed using Sanger sequencing. The 3UTR-Xba1-F (SEQ ID NO: 7) primer was used for base sequence analysis.

As a result of subculture at 37°C, it was confirmed that the length of the poly(A) sequence is not maintained in most colonies (Table 3). Meanwhile, when subculture is carried out at 25°C, it was confirmed that the length of the poly(A) sequence of all colonies is maintained (Table 4).

**[Table 3]**

| Maintenance of length of poly(A) sequence during subculture at 37°C | | | | |
|---|---|---|---|---|
| Colony # | Culture temperature 37°C | | | Maintenance of length in primary culture solution ±1 |
| | Primary | Secondary | Tertiary | |
| 3 | 122 | 16 | 16 | X |
| 5 | 122 | 60 | 60 | X |
| 6 | 125 | 70 | n/a | X |
| 7 | 122 | 124 | 122 | O |
| 15 | 122 | 60 | 62 | X |
| 22 | 122 | 123 | 122 | O |

**[Table 4]**

| Maintenance of length of poly(A) sequence during subculture at 25°C | | | | | |
|---|---|---|---|---|---|
| Colony # | Culture temperature 25°C | | | | Maintenance of length in primary culture solution ±1 |
| | Primary | Secondary | Tertiary | Quaternary | |
| 3 | 125 | 125 | 125 | 125 | ○ |
| 5 | 123 | 123 | 122 | 122 | ○ |
| 7 | 124 | 124 | 124 | 124 | ○ |
| 9 | 122 | 122 | 122 | 121 | ○ |
| 11 | 124 | 124 | 124 | 124 | ○ |
| 15 | 124 | 124 | 123 | 123 | ○ |
| 17 | 124 | 124 | 124 | 123 | ○ |
| 19 | 123 | 123 | 123 | 123 | ○ |
| 23 | 123 | 123 | 123 | 123 | ○ |

Through this, it was confirmed that culture at a temperature of 31°C or less during subculture is a suitable condition for maintaining the poly(A) sequence of a plasmid.

### Example 4. Confirmation of stability after preparation of cell line stock

In order to examine the ease of storage of cell lines constructed through transformation, the transformed cell line, of which the A length was confirmed to be 124 under a condition of 25°C in Example 3, was prepared in the form of glycerol stock and stored in a freezer at -70°C. The stocks stored in a frozen state were taken out and subjected to liquid culture in different test tubes at 16°C, 25°C, and 30°C, and then each plasmid was isolated and subjected to sequence analysis. The analyzed A lengths in the plasmids isolated from cultured *E. coli* were all 124, and it was confirmed that the A length is kept intact when a stock is prepared and then cultured.

**[Table 5]**

| Stock culture # | A length at various culture temperatures | | |
|---|---|---|---|
| | 16°C | 25°C | 30°C |
| 1 | 124 | 124 | 124 |
| 2 | 124 | 124 | 124 |
| 3 | 124 | 124 | 124 |
| 4 | 124 | 124 | 124 |
| 5 | 124 | 124 | 124 |
| 6 | 124 | 124 | 124 |

### Example 5. Confirmation of culture curves at various temperatures

The following experiment was conducted to confirm that culture under a condition of less than 16°C was not appropriate for process application.

The transformed cell lines DH5alpha and NEB stable, of which the A length was confirmed to be 124 under a condition of 25°C in Examples 2 and 3, were each prepared in the form of glycerol stock and stored in a freezer at -70°C. In order to confirm the culture curves at various temperatures, the stocks were taken out and cultured for 48 hours at 200 rpm under conditions of 13°C, 16°C, 19°C, 22°C, and 25°C, and the OD₆₀₀ was measured at predetermined times during culture. As a result of the experiment, it was confirmed that there is almost no cell growth at 13°C, which is less than 16°C (FIG. 5).

Based on the above description, it will be understood by those skilled in the art that the present invention may be implemented in a different specific form without changing the technical spirit or essential characteristics thereof. Therefore, it should be understood that the above embodiment is not limitative, but illustrative in all aspects. The scope of the present invention is defined by the appended claims rather than by the description preceding them, and therefore all changes and modifications that fall within metes and bounds of the claims or equivalents of such metes and bounds are therefore intended to be embraced by the claims.

### [Sequence List]

SEQ ID NO: 1 
ATTTTCATTGCCCGCGGAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAGTCTTCGATATCAAGCTTG
GC
SEQ ID NO: 2
   GCCAAGCTTGATATCGAAGACTTT
SEQ ID NO: 3 (pSKBS01) 
CCGTCGTGTAGATAACTACGATACGGGAGGGCTTACCATCTGGCCCCAG
TGCTGCAATGATACCGCGAGACCCACGCTCACCGGCTCCAGATTTATCAGCAA
TAAACCAGCCAGCCGGAAGGGCCGAGCGCAGAAGTGGTCCTGCAACTTTATCC
GCCTCCATCCAGTCTATTAATTGTTGCCGGGAAGCTAGAGTAAGTAGTTCGCCA
GTTAATAGTTTGCGCAACGTTGTTGCCATTGCTACAGGCATCGTGGTGTCACGC
TCGTCGTTTGGTATGGCTTCATTCAGCTCCGGTTCCCAACGATCAAGGCGAGTT
ACATGATCCCCCATGTTGTGCAAAAAAGCGGTTAGCTCCTTCGGTCCTCCGATC
GTTGTCAGAAGTAAGTTGGCCGCAGTGTTATCACTCATGGTTATGGCAGCACTG
CATAATTCTCTTACTGTCATGCCATCCGTAAGATGCTTTTCTGTGACTGGTGAGT
ACTCAACCAAGTCATTCTGAGAATAGTGTATGCGGCGACCGAGTTGCTCTTGCC
 
CGGCGTCAATACGGGATAATACCGCGCCACATAGCAGAACTTTAAAAGTGCTCA
TCATTGGAAAACGTTCTTCGGGGCGAAAACTCTCAAGGATCTTACCGCTGTTGA
GATCCAGTTCGATGTAACCCACTCGTGCACCCAACTGATCTTCAGCATCTTTTA
CTTTCACCAGCGTTTCTGGGTGAGCAAAAACAGGAAGGCAAAATGCCGCAAAA
AAGGGAATAAGGGCGACACGGAAATGTTGAATACTCATACTCTTCCTTTTTCAAT
ATTATTGAAGCATTTATCAGGGTTATTGTCTCATGAGCGGATACATATTTGAATG
TATTTAGAAAAATAAACAAATAGGGGTTCCGCGCACATTTCCCCGAAAAGTGCC
ACCTGACGTCTAAGAAACCATTATTATCATGACATTAACCTATAAAAATAGGCGT
ATCACGAGGCCCTTTCGTC
SEQ ID NO: 4 (M13-Forward)
   GTAAAACGACGGCCAGT
SEQ ID NO: 5 (M13-Reverse)
   CAGGAAACAGCTATGAC
SEQ ID NO: 6 (Atail-seq-R)
   TGGATAACCGTATTACCGCC
SEQ ID NO: 7 (3UTR-Xba1-F)
   TCCTCTAGAAGCTGCTTGTCAATTTCTA

## Claims

1. A method for stably maintaining a poly(A) tail in a plasmid, the method comprising:
culturing a cell line transformed with a plasmid containing a sequence encoding a poly(A) tail at a temperature condition of 31°C or less.

2. The method according to claim 1, wherein the culturing step includes (1) recovering the cell line at a temperature condition of 31°C or less.

3. The method according to claim 2, which comprises (2) culturing the cell line on a solid medium at a temperature condition of 31°C or less to form a colony after the recovery step of (1).

4. The method according to claim 3, wherein the culture further includes (3) culturing the colony formed by the solid medium culture of (2) in a liquid medium at a temperature condition of 31°C or less.

5. The method according to claim 4, wherein the step of culturing the colony in a liquid medium includes performing subculture.

6. The method according to any one of claims 1 to 4, wherein the temperature condition is 16°C to 31°C.

7. The method according to any one of claims 1 to 4, wherein the temperature condition is 19°C to 25°C.

8. The method according to claim 1, wherein the cell line is *E*. *coli.*

9. The method according to claim 1, wherein the transformation includes introducing a plasmid containing a sequence encoding a poly(A) tail into a cell line using heat shock or electroporation.

10. The method according to claim 9, wherein the introduction is performed at a temperature of 37°C to 42°C.

11. The method according to claim 1, wherein the method increases stability of a poly(A) tail in a plasmid compared to a method comprising culturing a cell line transformed with a plasmid containing a sequence encoding a poly(A) tail at a temperature condition of 37°C or more.

12. The method according to claim 1, wherein the poly(A) tail contains 20 to 400 adenines (A).

13. A method for mass-producing a plasmid for mRNA production, the method comprising:
culturing a cell line transformed with a plasmid containing a sequence encoding an mRNA containing a poly(A) tail at a temperature condition of 31°C or less.

14. A method for producing an mRNA containing a poly(A) tail, the method comprising:
culturing a cell line transformed with a plasmid containing a sequence encoding an mRNA containing a poly(A) tail at a temperature condition of 31°C or less; and
performing transcription using the plasmid of the cell line as a template.
